# EUROPÄISCHE PATENTSCHRIFT

(11) **EP 1 377 212 B1**
(45) Veröffentlichungstag und Bekanntmachung des Hinweises auf die Patenterteilung: **11.05.2005**
(21) Anmeldenummer: 02706502.8
(22) Anmeldetag: 12.03.2002
(51) Int. Cl.: A61B 5/053, A61B 5/0408

(54) **MEDIZINISCHE BANDELEKTRODE**
MEDICAL STRIP ELECTRODE
ELECTRODE A BANDE MEDICALE

(30) Priorität: 13.03.2001 AT 3922001
(43) Veröffentlichungstag der Anmeldung: 07.01.2004
(73) Patentinhaber: CNSystems Medizintechnik GmbH, 8020 Graz (AT); Nessler Medizintechnik GmbH, 6020 Innsbrück (AT)
(72) Erfinder: FORTIN, Jürgen, A-8020 Graz (AT); NESSLER, Winfried, 81739 München (DE); NESSLER, Bernhard, A-6020 Innsbruck (AT); SKRABAL, Falko, A-8043 Graz (AT)
(74) Vertreter: Häupl, Armin, Dipl.-Ing.
(86) Internationale Anmeldenummer: PCT/AT2002/000081
(87) Internationale Veröffentlichungsnummer: WO 2002/071940

(56) Entgegenhaltungen:
- FR-A- 2 186 828
- US-A- 4 562 843
- MIYAMOTO Y ET AL: "Continuous determination of cardiac output during exercise by the use of impedance plethysmography" MEDICAL & BIOLOGICAL ENGINEERING & COMPUTING, SEPT. 1981, UK, Bd. 19, Nr. 5, Seiten 638-644, XP002204394 ISSN: 0140-0118
- WOLTJER H H ET AL: "OPTIMALISATION OF THE SPOT ELECTRODE ARRAY IN IMPEDANCE CARDIOGRAPHY" MEDICAL AND BIOLOGICAL ENGINEERING AND COMPUTING, PETER PEREGRINUS LTD. STEVENAGE, GB, Bd. 34, Nr. 1, 1996, Seiten 84-87, XP000549096 ISSN: 0140-0118

## Beschreibung

Die vorliegende Erfindung betrifft medizinische Elektroden, insbesondere Impedanz-Kardiografie- (IKG-) Elektroden.

Die Impedanz-Kardiografie ist ein relativ neues Gebiet der medizinischen Diagnostik und beruht auf der Messung des Widerstands (Impedanz) des menschlichen Körpers für elektrischen Strom bei Anlegung eines Wechselfeldes. Aus der berechneten Impedanzänderung bei Durchleiten eines definierten Wechselstroms durch den Brustkorb eines Patienten und Messung von Spannungsänderungen aufgrund von Änderungen des Blutstroms innerhalb des Feldes lassen sich verschiedene Kenngrößen, wie z.B. Herzschlagvolumen, Herzdurchsatz, systemischer Gefäßwiderstand, Geschwindigkeit und Beschleunigung der Blutzirkulation, Herzpumpleistung, Herzkontraktion usw., ableiten.

Nach dem Stand der Technik sind verschiedene Messanordnungen für die Impedanz-Kardiografie bekannt. Ein Verfahren verwendete mittels Lungenarterien-Katheter eingeführte innenliegende Elektroden, wurde jedoch von weitaus praktischeren nicht-invasiven Methoden, die kein Kathetersetzen erfordern, abgelöst, wovon eines darin besteht, am Hals und unterhalb des Thorax (unter dem Rippenbogen) je zwei ringförmige Elektroden, z.B. aus Silber- oder Aluminium-Bändern, rund um den gesamten Körperumfang an diesen Stellen anzubringen (z.B. aufzukleben).

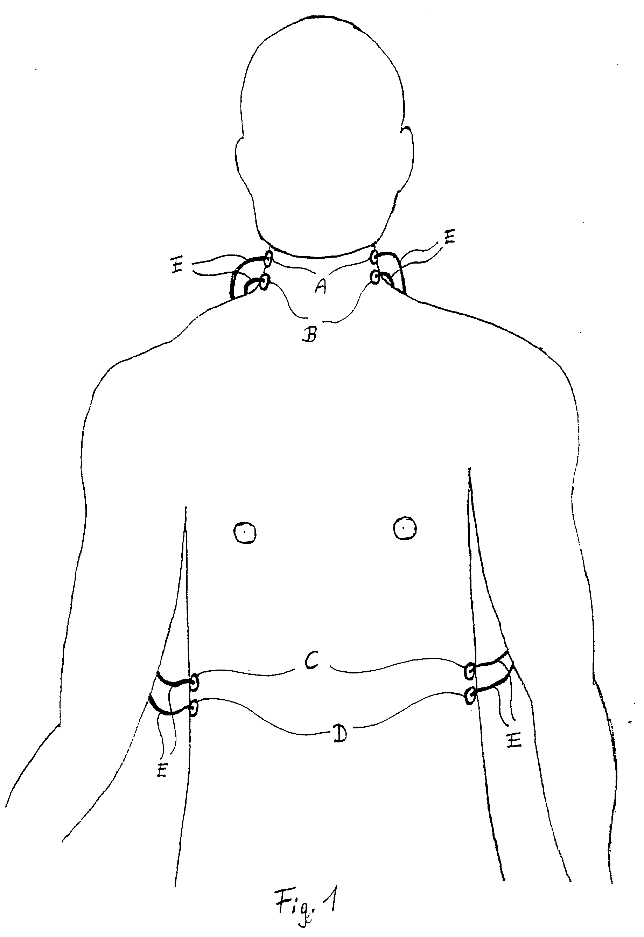

Dies ist jedoch unpraktisch, weil:
- der Körper des Patienten beim Anbringen der Elektroden unvermeidlich bewegt werden muss, was im Falle von Verletzungen ein erhebliches Risiko für die betreffende Person darstellt;
- die Elektroden bei Bewegungen des Patienten leicht verrutschen und sich von der Haut ablösen können;
- die Atmung des Patienten durch die zirkulären Elektroden behindert und erschwert wird;
- die Elektroden in der richtigen, dem Körperumfang an der jeweiligen Stelle entsprechenden Länge von einem Endlosband abgeschnitten werden müssen;
- an die Metallbänder, sobald sie in der richtigen Länge vorliegen, noch in einem gesondertem Schritt die elektrischen Anschlüsse angeklebt oder -gelötet werden müssen,
- kein ausreichend konstantes homogenes Feld aufgebaut werden kann, um auch geringfügige Impedanzänderungen zuverlässig messen zu können, da der Abstand zwischen den beiden Bändern eines Paares nicht definiert und nicht konstant ist; und
- die Kosten für das Elektrodenmaterial und der Arbeitsaufwand unwirtschaftlich hoch sind.
   Eine Modifikation dieses Verfahrens sieht anstelle der Ringelektroden diskrete Elektroden am Hals und unter dem Rippenbogen vor. Dabei kommen beispielsweise herkömmliche, kreisrunde EKG-Elektroden zum Einsatz. Dabei werden in Körperachsenrichtung paarweise nebeneinander (d.h. beim stehend gedachten Patienten: übereinander) liegende Elektroden angebracht. Auch hier ist ein Elektrodenpaar am Hals und zumindest eines unterhalb des Thorax vonnöten, zumeist werden aber jeweils zwei einander auf den beiden Seiten des Körpers gegenüberliegende Elektrodenpaare (also 8 Elektroden) angebracht, um die Homogenität des Feldes zu verbessern. Die "äußeren" Elektroden (d.h. die oberen Hals- und die unteren Rippenbogenelektroden) dienen zum Feldaufbau, während die jeweils innerhalb des Feldes liegenden zur Potentialmessung herangezogen werden (siehe Fig. 1 und die später folgenden zugehörigen Erläuterungen).
   Dieses Verfahren liefert bessere Ergebnisse als die Methode unter Verwendung der Ringelektroden und ist auch einfacher und kostengünstiger durchzuführen, da der Körper des Patienten zur Aufbringung der Elektroden nicht bewegt zu werden braucht, die Elektroden vorgefertigt sind, d.h. nicht an den Körper angepasst (geschnitten) werden müssen, und in Form von handelsüblichen EKG-Elektroden leicht verfügbar sind.
   Der Nachteil dieser Ausführungsform der Impedanz-Kardiografie liegt jedoch genau in dieser Vorfertigung, genauer gesagt in der Elektrodengeometrie. Durch die punktuelle Stromzufuhr kann auch in diesem Fall kein ausreichend homogenes Feld erzeugt werden, um bei geringfügigen Impedanzänderungen detektierbare und signifikante Signale zu erhalten. Ein großer Anteil diagnostisch wertvoller Informationen ist vom unvermeidlichen Hintergrundrauschen nicht unterscheidbar. Auch leidet durch den Einsatz von Paaren diskreter Elektroden die Reproduzierbarkeit, da der Abstand zwischen den Elektroden eines Paares nicht bei allen Messungen derselbe ist, was zu Schwankungen der Messergebnisse führt. Selbst bei Verbindung der beiden Elektroden eines Paares (z.B. durch Vorfertigung auf einer vorgestanzten Folie in Form einer "8") bleibt die Möglichkeit bestehen, dass diese Acht nicht parallel zur Körperachse aufgeklebt wird, was die Reproduzierbarkeit der Messergebnisse beeinträchtigt.

FR-A-2 186 828 offenbart eine medizinische Elektrode mit einem länglich geformten Träger, zwei Kontaktstreifen und eine Schutzabdeckung.

Ziel der Erfindung ist daher die Bereitstellung von neuen Elektroden, mit denen die oben beschriebenen Nachteile des Standes der Technik überwunden werden können.

Dieses Ziel wird erfindungsgemäß mit einer medizinischen Elektrode zur Messung des elektrischen Widerstands des Körpers von Patienten, insbesondere einer Impedanz-Kardiografie- (IKG-) Elektrode gemäß Anspruch 1 erreicht.

Bei Verwendung derartiger erfindungsgemäßer Elektroden ist ein homogenes elektrisches Feld gewährleistet, da die Stromzufuhr nicht punktuell, sondern über eine längere Strecke (quer zur Körperachse) erfolgt, ohne dass zum Aufbringen der Elektroden der Patient bewegt zu werden braucht. Das eigentliche Elektrodenmaterial ist ein Verbund einer Aluminiumfolie mit einem hautverträglichen, elektrisch leitenden Kleber, d.h. das Elektrodenmaterial klebt direkt auf der Haut, wodurch der Kontakt mit dem Körper des Patienten über die gesamte Länge sichergestellt ist, so dass Ablösungen vermieden werden.

Durch Vorsehen einer derartigen zweigeteilten Elektrode ist zudem ein konstanter Abstand zwischen den beiden streifenförmigen Kontakten garantiert, was für bestmögliche Reproduzierbarkeit der Messungen sorgt. Weiters brauchen zur Erzeugung des elektrischen Felds anstelle von acht Elektroden nach dem Stand der Technik nur zwei oder drei erfindungsgemäße Elektroden am Körper angebracht zu werden, da sich die Bauchwie auch die Halselektrode von einer Körperseite zur anderen erstrecken können, was folglich auch die Anzahl an Anschlüssen und somit Kabeln verringert und dadurch die Handhabbarkeit weiter verbessert.

Die zwei Kontaktstreifen verlaufen - außer im Bereich des Übergangs zu den Anschlusslaschen - bevorzugt im Wesentlichen parallel in einem Abstand von 15 bis 50 mm, vorzugsweise 20 bis 40 mm, insbesondere 25 bis 30 mm, und weisen eine Breite von 3 bis 10 mm, vorzugsweise 4 bis 7 mm, insbesondere 5 mm, auf. Die Länge der Kontaktstreifen liegt dabei bevorzugt in einem Bereich von 50 bis 500 mm, vorzugsweise 100 bis 400 mm, noch bevorzugter 150 bis 300 mm, insbesondere bei etwa 200 mm.

Der Mindestabstand zwischen den Kontaktstreifen ist erforderlich, damit es zu keinen gegenseitigen Störungen und Beeinträchtigungen des Wechselfeldes kommt. Der erfindungsgemäße Höchstabstand ergibt sich aus Kostengründen, da ein größerer Abstand die Herstellungskosten unnötig erhöhen würde. Sowohl bei Länge als auch Breite der Kontaktstreifen wurde ein Kompromiss zwischen möglichst großer Kontaktlänge bzw. -fläche und möglichst geringen Herstellungskosten gefunden.

Der Träger besteht üblicherweise aus elektrisch nicht leitendem, einseitig klebendem Kunststoffschaum. Auch der einseitig darauf vorgesehene elektrisch nicht leitende Kleber ist vorzugsweise hautverträglich, da er während der Messung in direktem Kontakt mit der Haut des Patienten steht.

Die Geometrie der Anschlusslaschen entspricht in einer bevorzugten Ausführungsform jener von genormten, handelsüblichen elektrischen Anschlüssen, z.B. für einen Anschlussclip von Neutralelektroden für die HF-Chirurgie, damit die Anfertigung spezieller Anschlüsse für die erfindungsgemäßen Elektroden entfallen kann, was wiederum Kosten spart und die Handhabbarkeit der Elektroden weiter verbessert.

Die vorliegende Erfindung wird nachstehend unter Bezugnahme auf die beiliegenden Zeichnungen näher erklärt, worin:
Fig. 1 schematisch eine Impedanz-Kardiografie-Messanordnung unter Verwendung von acht Elektroden nach dem Stand der Technik zeigt;
Fig. 2 schematisch eine ähnliche Impedanz-Kardiografie-Messanordnung wie in Fig. 1, jedoch unter Verwendung von nur drei erfindungsgemäßen Elektroden zeigt; und
Fig. 3 eine Konstruktionszeichnung einer bevorzugten Ausführungsform der erfindungsgemäßen Elektrode zeigt.

In Fig. 1 ist eine schematische Abbildung des Oberkörpers eines Patienten zu sehen, der einer Impedanz-Kardiografie-Messung unterzogen werden soll. An seinem Hals sowie unterhalb des Rippenbogens sind jeweils vier, paarweise auf beiden Seiten des Körpers angeordnete Elektroden A, B, C und D nach dem Stand der Technik mit zugehörigen Anschlusskabeln E angebracht. Als Elektroden dienen beispielsweise herkömmliche kreisrunde EKG-Elektroden, deren leitende Kontaktfläche üblicherweise die Form eines Kreises mit einem Durchmesser von etwa 10 bis 12 mm besitzt.

Die beiden obersten und untersten Elektroden in Fig. 1 (die Elektroden A und D) dienen dabei zur Erzeugung des elektrischen Feldes über den gesamten Brustkorb des Patienten, während die jeweils innenliegenden der Elektrodenpaare (die Elektroden B und C) zur Messung der Impedanz herangezogen werden.

Die Stromzufuhr erfolgt nach dem Stand der Technik somit punktuell, weswegen es nicht in ausreichendem Maße gelingt, ein homogenes elektrisches Wechselfeld zwischen den (in diesem Fall vier) Punktelektroden aufzubauen, um empfindliche Messungen vornehmen zu können.

In der medizinischen Praxis wird (z.B. bei Verwendung von Systemen mit den Markennamen BioZ® und BioZ.com® der Fa. CardioDynamics) ein Wechselstrom mit einer Frequenz von 70 kHz und einer Stärke von 2,5 mA zum Aufbau des Feldes eingespeist. Erlaubt sind gemäß ISO- oder EN-Normen (Nr. 60-601-1) maximal 4 mA Stromstärke, für BF- ("body flow") Zertifizierungen. Die Nachweisgrenze der Potentialmessung liegt gemäß dem Stand der Technik nach Verstärkung, Integrieren und digitaler Verarbeitung der Signale in der Größenordnung von 0,1 bis 1 µV.

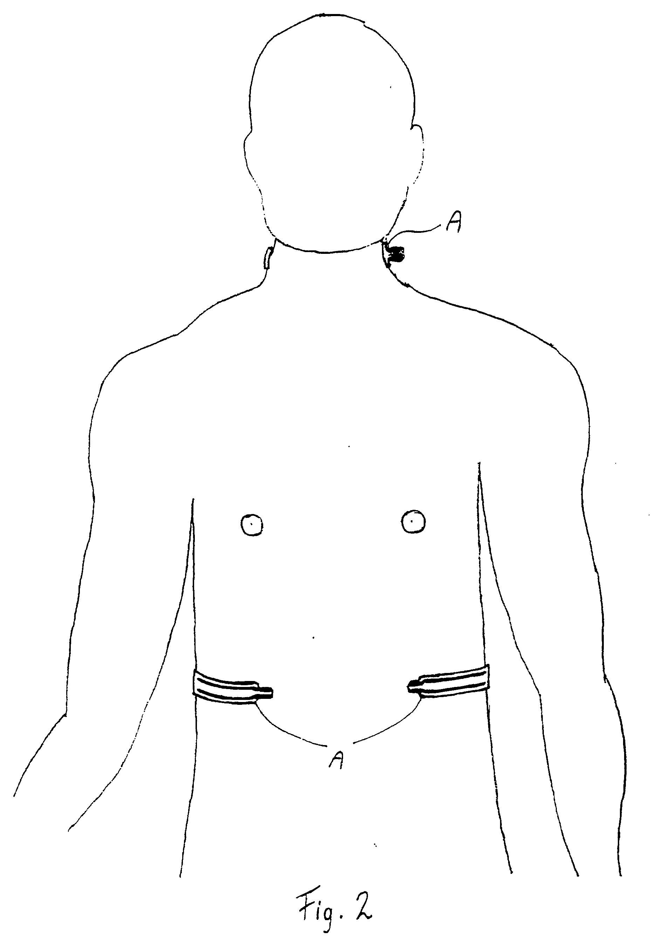

Fig. 2 zeigt im Vergleich dazu schematisch eine Messanordnung unter Verwendung der erfindungsgemäßen Elektroden. In der Figur ist am Nacken (in der Zeichnung sind daher nur die Enden zu erkennen) eine erfindungsgemäße Elektrode A vorgesehen, die von einer Seite des Halses zur anderen reicht, weswegen keine zweite Halselektrode erforderlich ist. Unterhalb des Thorax sind zwei weitere, baugleiche Elektroden A auf gleicher Höhe angebracht, d.h. aufgeklebt. In dieser Skizze ist, besonders bei den beiden unteren Elektroden, die Teilung des Elektrodenmaterials in zwei Kontaktstreifen schematisch angedeutet, obwohl diese in der Praxis natürlich vom Träger verdeckt wären.

Die Kabelanschlüsse der Elektroden kleben nicht am Körper, was besonders im Fall der Halselektrode gut erkennbar ist, und werden für die Messung über (nicht dargestellte) Standard-Anschlussclips, wie sie z.B. für HF-chirurgische Neutralelektroden zum Einsatz kommen, an Stromquelle bzw. Messgerät angeschlossen. Somit braucht für die erfindungsgemäßen Elektroden kein eigener Anschlussclip entwickelt zu werden.

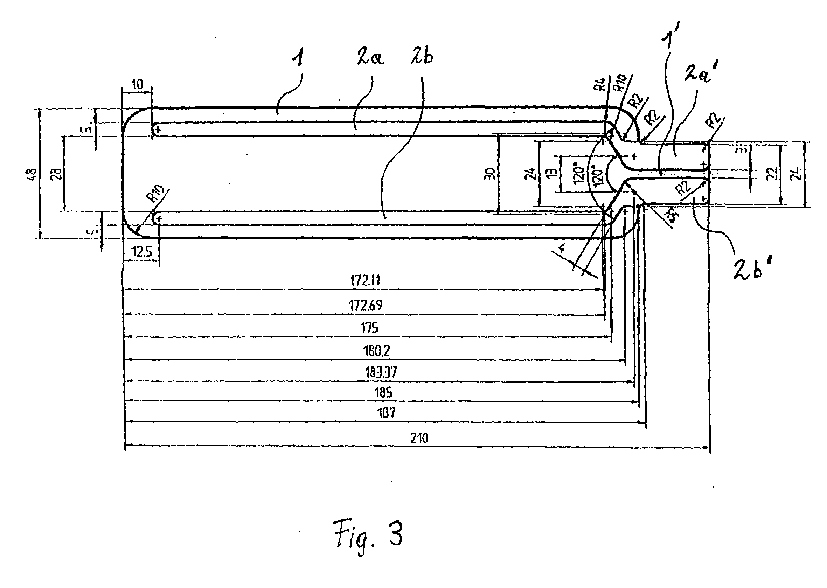

Wie aus den Fig. 1 und 2 zu entnehmen ist, sind die Elektrodenflächen der Kontaktstreifen gemäß vorliegender Erfindung gegenüber dem Stand der Technik deutlich vergrößert und verlaufen konstruktionsbedingt (siehe Fig. 3) auf alle Fälle parallel und in konstantem Abstand zueinander. Außerdem ist es für das medizinische Personal (z.B. Krankenschwestern) einfacher, länglich geformte Elektroden aus Fig. 2 richtig, d.h. im rechten Winkel zur Körperachse und auf gleicher Höhe, aufzukleben als die viel kleineren EKG-Elektroden aus Fig. 1 in der korrekten Position anzubringen (parallel zur Körperachse und auf gleicher Höhe), selbst wenn diese zu einer "8" verbunden wären (wofür ein eigener zusätzlicher Herstellungsschritt vonnöten wäre und die herkömmlichen EKG-Elektroden nicht mehr direkt einsetzbar wären).

Es ist gemäß vorliegender Erfindung auch möglich nur zwei Elektroden, d.h. eine am Hals und eine am Rippenbogen, zu verwenden. Die untere Elektrode kann dabei entweder die gleiche Länge aufweisen wie die Halselektrode oder aber länger, z.B. etwa 500 mm lang, ausgeführt sein, um sich quer über den ganzen Bauch zu erstrecken. Erstere Konfiguration ist im Hinblick auf ein möglichst homogenes Wechselfeld nicht bevorzugt, letztere aus wirtschaftlichen Gründen, da es weitaus kostengünstiger ist, nur eine (kürzere) Bauweise der erfindungsgemäßen Elektrode zu erzeugen. Fig. 2 stellt somit den erfindungsgemäß bevorzugten Kompromiss zwischen Feldhomogenität und Herstellungskosten unter Verwendung von drei Elektroden A mit einer Länge von rund 200 mm dar, was etwa dem halben Halsumfang eines Erwachsenen entspricht.

In Fig. 3 ist diese bevorzugte Ausführungsform der vorliegenden Erfindung als Konstruktionszeichnung in Draufsicht wiedergegeben. Auf einem Träger 1 mit einer Länge von 210 mm sind zwei Kontaktstreifen 2a, 2b angeordnet. Der Träger 1 besteht vorzugsweise aus einem Schaumstoff, wie er für medizinische Elektroden üblicherweise zur Anwendung kommt, womit das Material kostengünstig herzustellen, weich und biegsam ist, um sich den Körperkonturen gut anpassen zu können. Die (dem Betrachter zugewandte) Oberseite des Trägers ist klebend, d.h. mit einem nicht elektrisch leitenden, vorzugsweise hautverträglichen, Kleber, z.B. Gel, versehen, und die Kontaktstreifen 2a, 2b sind über diesen Kleber mit dem Träger 1 so fest verbunden, dass sie sich beim Hantieren mit der Elektrode und besonders beim Abziehen derselben von der Haut nicht vom Träger 1 ablösen können.

Die Kontaktstreifen 2a, 2b bestehen in diesem Fall aus einem Verbundmaterial aus einer Aluminium- und einer stabilisierenden Kunststofffolie, im Verbund mit einem hautverträglichen, elektrisch leitenden Kleber auf der Aluminium-Seite, die zum Festkleben auf der Haut dient, während die andere, die Kunststoff-Seite der Verbundfolie fest mit dem Träger 1 verklebt ist.

Die Gesamtlänge des Trägers 1 beträgt 210 mm, jene der Kontaktstreifen 2a, 2b in dieser Ausführungsform 200 mm, d.h. der Träger überragt die Kontaktstreifen 2a, 2b einseitig um 10 mm, was ein Ablösen der Kontaktstreifenenden von der Haut - zusätzlich zur Klebewirkung der Kontaktstreifen selbst - verhindert.

Die Gesamtbreite des Trägers 1 beträgt 48 mm, die Breite der Kontaktstreifen 2a, 2b beträgt über den Großteil ihrer Länge 5 mm. Eine Breite der Kontaktstreifen 2a, 2b von weniger als 3 mm ist nicht bevorzugt, da sich die Homogenität des Feldes bei zu geringer Breite der Kontaktstreifen unannehmbar verschlechtert, während eine größere Breite als etwa 10 mm die Herstellungskosten unnötig erhöht, da sie nicht mehr zur Feldstabilisierung beiträgt. Der bevorzugte Bereich liegt bei 4 bis 7 mm, insbesondere 5 bis 6 mm haben sich als optimal herausgestellt.

Der Träger 1 überragt die Kontaktstreifen 2a, 2b zum Längsrand der Elektrode hin um 5 mm, was wiederum zur zusätzlichen Fixierung der Kontaktstreifen an der Haut dient.

Über den Großteil der Elektrodenlänge verlaufen die Kontaktstreifen 2a, 2b zueinander parallel in einem definierten Abstand von (in dieser Ausführungsform) 28 mm. Der Mindestabstand für gegenseitige Störungsfreiheit der Kontakte beträgt 15 bis 20 mm, der aus wirtschaftlichen Überlegungen sinnvolle Maximalabstand etwa 50 mm. Ein Abstand von 28 bis 30 mm wurde als optimaler Kompromiss zwischen Störungsfreiheit und Materialkosten ermittelt.

An einem Ende gehen sowohl der Träger 1 als auch die Kontaktstreifen 2a und 2b in jeweilige Anschlusslaschen 1', 2a' bzw. 2b' über, wobei sich die Breite des Trägers 1 (von 48 mm auf 22 mm) verringert, während sich die Kontaktstreifen 2a, 2b (von 5 mm auf 9,5 mm) verbreitern und sich ihr Abstand zueinander (von 30 mm auf 3 mm) verringert. Dadurch kann an der so gebildeten Lasche ein herkömmlicher Anschlussclip angebracht werden, die Konstruktion eines speziellen Clips somit kostensenkend entfallen.

Die Kontaktstreifen sind im Bereich der Anschlusslaschen nicht klebend, um ein Verkleben des Anschlussclips während des Messung zu verhindern.

Die in Fig. 3 gezeigte Oberfläche von Träger 1 und Kontaktstreifen 2a, 2b ist während der Lagerung durch eine (nicht dargestellte) herkömmliche Abziehfolie geschützt, um die Kontakte und die Klebeflächen vor Verschmutzung oder Beschädigung zu bewahren. Diese Folie ist vor Gebrauch einfach zu entfernen.

Unter Verwendung derartiger erfindungsgemäßer Elektroden kann ein gegenüber dem Stand der Technik wesentlich homogeneres und stabileres Wechselfeld über den Brustkorb des Patienten aufgebaut werden, wodurch die Empfindlichkeit, Reproduzierbarkeit und Genauigkeit der Messungen deutlich erhöht werden.

Beispielsweise ist es mit der in Fig. 2 dargestellten Messanordnung möglich, mit einer Frequenz des Wechselfeldes von nur 40 kHz und einer Stromstärke von nur 350 µA zu arbeiten. Dadurch kann die Nachweisgrenze der gemessenen Spannungssignale in den Bereich von 0,01 µV gesenkt werden, wobei die Messwerte innerhalb von wenigen Sekunden zugänglich sind, während nach dem Stand der Technik bis zu einer Minute zugewartet werden muss, um ein eindeutiges Signal zu erhalten.

Darüber hinaus ist eine derartige Messanordnung unter Verwendung der erfindungsgemäßen Elektroden für "cardiac flow" (CF-) Anwendungen zertifizierbar, wofür gemäß ISO- oder EN-Normen (Nr. 60-601-1) maximal 0,4 mA Stromstärke eingespeist werden dürfen, was bedeutet, dass mit Elektroden der vorliegenden Erfindung beispielsweise auch während Operationen am offenen Herzen kontinuierlich die Impedanz gemessen werden kann.

Die vorliegende Erfindung stellt somit neue medizinische Elektroden, insbesondere Elektroden für die Impedanz-Kardiografie, bereit, die gegenüber dem Stand der Technik folgende Vorteile bieten:
1) der Körper des Patienten braucht nicht bewegt zu werden, um die Elektroden aufzukleben;
2) die Elektroden sind vorgefertigt, d.h. kein Zurechtschneiden der Elektroden bzw. Anlöten von Kontakten ist erforderlich;
3) es sind nur zwei oder drei erfindungsgemäße Elektroden vonnöten, was die Handhabung erleichtert und die Aufbringung beschleunigt;
4) das Aufkleben der Elektroden in der richtigen Anordnung, d.h. parallel zueinander bzw. auf der gleichen Höhe und im rechten Winkel zur Körperachse, ist deutlich einfacher, was reproduzierbarere Messungen ermöglicht;
5) die Kontaktstreifen der erfindungsgemäßen Elektroden haften direkt am Körper des Patienten, so dass ein Verrutschen verhindert wird;
6) ein stabileres, homogenes elektrisches Wechselfeld kann aufgebaut werden, was weitaus genauere Messungen ermöglicht;
7) der Messbereich kann um zumindest eine Zehnerpotenz gesenkt werden, wodurch nicht nur die Genauigkeit zunimmt, sondern auch "CF"-Zertifizierung für Messungen auch während Operationen am offenen Herzen erzielbar ist;
8) die Kosten können deutlich gesenkt werden, da statt Ag billiges Al als Elektrodenmaterial dient, in bevorzugten Ausführungsformen der Erfindung nur eine Elektrodenform hergestellt zu werden braucht, die Haut nicht mit leitendem Gel vorbehandelt werden muss und herkömmliche Anschlussclips verwendet werden können.

Aufgrund derartig vereinfachter Handhabbarkeit, gesenkter Herstellungskosten und exzellenter Reproduzierbarkeit der Messungen besteht kein Zweifel an der gewerblichen Anwendbarkeit der erfindungsgemäßen Elektroden.

## Patentansprüche

1. Medizinische Elektrode zur Messung des elektrischen Widerstands des Körpers von Patienten, insbesondere Impedanz-Kardiografie- (IKG-) Elektrode, bestehend aus folgenden Komponenten:
einem elektrisch nicht leitenden, einseitig klebenden Träger (1), der länglich geformt ist und an einem Ende in eine Anschlusslasche (1') zum Verbinden der Elektrode mit elektrischen Anschlüssen übergeht;
zwei voneinander elektrisch isolierten, im Wesentlichen parallel verlaufenden Kontaktstreifen (2a, 2b) aus elektrisch leitender Al-Verbundfolie als Elektrodenmaterial, die an der klebenden Seite des Trägers (1) auf diesen aufgeklebt sind, **dadurch gekennzeichnet, daß** die Kontaktstreifen an ihrer vom Träger (1) abgewandten Oberfläche unter Aussparung der Anschlusslaschen (2a', 2b') eine Verbundstruktur mit einem hautverträglichen elektrisch leitenden Kleber bilden und am Anschlussende des Trägers (1) ebenfalls in jeweilige Anschlusslaschen (2a', 2b') übergehen, wobei die Länge der Kontaktstreifen (2a, 2b) im Bereich von 50 bis 600 mm, vorzugsweise 100 bis 400 mm, noch bevorzugter 150 bis 300 mm, insbesondere bei etwa 200 mm, liegt; sowie gegebenenfalls
einer abziehbaren Schutzabdeckung für die in Verwendung mit dem Körper des Patienten in Kontakt kommenden klebenden Oberflächen des Trägers (1) und der Kontaktstreifen (2a, 2b).

2. Elektrode nach Anspruch 1, **dadurch gekennzeichnet, dass** die zwei Kontaktstreifen (2a, 2b) mit Ausnahme des Übergangs zu den Anschlusslaschen (2a', 2b') im Wesentlichen parallel in einem Abstand von 15 bis 50 mm, vorzugsweise 20 bis 40 mm, insbesondere 28 bis 30 mm, verlaufen und eine Breite von 3 bis 10 mm, vorzugsweise 4 bis 7 mm, insbesondere 5 bis 6 mm, aufweisen.

3. Elektrode nach Anspruch 1 oder 2, **dadurch gekennzeichnet, dass** der Abstand zwischen den äußeren Rändern der Kontaktstreifen (2a, 2b) und dem äußeren Rand des Trägers (1) mit Ausnahme des Bereichs der Anschlusslaschen (1', 2a', 2b') 1 bis 20 mm, vorzugsweise 3 bis 15 mm, besonders bevorzugt 4 bis 12 mm, beträgt, wobei der Abstand an den Längsrändern der Elektrode insbesondere etwa 5 mm und jener am den Anschlusslaschen (1', 2a', 2b') gegenüberliegenden Ende der Elektrode insbesondere etwa 10 mm beträgt.

4. Elektrode nach einem der Ansprüche 1 bis 3, **dadurch gekennzeichnet, dass** der Träger (1) aus elektrisch nicht leitendem, einseitig klebendem Kunststoffschaum besteht.

5. Elektrode nach Anspruch 4, **dadurch gekennzeichnet, dass** der Kleber an der einen Seite des Trägers (1) ebenfalls hautverträglich ist.

6. Elektrode nach einem der vorangegangenen Ansprüche, **dadurch gekennzeichnet, dass** die Geometrie der Anschlusslaschen (1', 2a', 2b') jener von genormten, handelsüblichen elektrischen Anschlüssen, z.B. für einem Anschlussclip von Neutralelektroden für die HF-Chirurgie, entspricht.

## Claims

1. A medical electrode for measuring the electrical resistance of a patient's body, particularly an impedance cardiography (ICG) electrode consisting, of the following components:
an electrically non-conducting, unilaterally adhesive support (1) of elongated shape, which forms, at one end, a terminal lug (1') for connecting the electrode with electrical connectors;
two contact strips (2a, 2b) extending in substantially parallel relation, which strips are electrically isolated from each other, are made of an electrically conducting Al-composite film as an electrode material and are bonded to the support (1) at its adhesive side,
**characterized in that** the contact strips form - at their surfaces facing away from the support (1) and sparing the terminal lugs (2a', 2b') - a composite structure with a non-irritant electrically conducting adhesive and also form respective terminal lugs (2a', 2b') at the terminal end of the support (1), the length of the contact strips (2a, 2b) being in the range of 50 to 600 mm, preferably 100 to 400 mm, more preferably 150 to 300 mm, in particular about 200 mm;
and optionally a releasable protective cover for the adhesive surfaces of the support (1) and the contact strips (2a, 2b), which, in use, contact the patient's body.

2. The electrode according to claim 1, **characterized in that**, except for the transition region to the terminal lugs (2a', 2b'), the two contact strips extend in substantially parallel relation, spaced by a distance of 15 to 50 mm, preferably 20 to 40 mm, particularly 28 to 30 mm, and have a width of 3 to 10 mm, preferably 4 to 7 mm, particularly 5 to 6 mm.

3. The electrode according to claim 1 or 2, **characterized in that**, except for the region of the terminal lugs (1', 2a', 2b'), the distance between the outer edges of the contact strips (2a, 2b) and the outer edge of the support is 1 to 20 mm, preferably 3 to 15 mm, particularly 4 to 12 mm, the distance particularly being about 5 mm at the longitudinal edges of the electrode and particularly being about 10 mm at the end of the electrode opposite to the terminal lugs (1', 2a', 2b').

4. The electrode according to any of claims 1 to 3, **characterized in that** the support (1) consists of electrically non-conducting, unilaterally adhesive plastic foam.

5. The electrode according to claim 4, **characterized in that** the adhesive on the one side of the support (1) is also non-irritant.

6. The electrode according to any of the preceding claims, **characterized in that** the geometries of the terminal lugs (1', 2a', 2b') correspond to those of standardized, commercially available electrical connectors, e.g. for connecting clips of neutral electrodes used in HF surgery.

## Revendications

1. Electrode médicale pour mésurer la résistance électrique du corps d'un patient, en particulier une électrode de cardiographie d'impédance (CGI), étant composée des composants suivants:
un support (1) de forme allongée, électriquement non conducteur et adhésif sur une face, qui forme/devient une patte de connexion (1') pour relier l'électrode à des connexions électriques;
deux bandes de contact (2a, 2b) électriquement isolées l'une de l'autre et s'étandant en substance parallèlement, faites d'une feuille composée d'Al comme matière d'électrode et étant collées au support (1) sur sa face adhésive,
**caractérisée en ce que** les bandes de contact forment aux surfaces non tournées vers le support, en omittant leurs pattes de connexion (2a', 2b'), une structure composée avec un adhésif non irritant et électriquement conducteur et forment, elles aussi, respectivement des pattes de connexion (2a', 2b') au bout de connexion du support (1), la longueur des bandes de contact (2a, 2b) étant comprise entre 50 à 600 mm, de préférence entre 100 et 400 mm, plus préférentiellement encore entre 150 et 300 mm, en particulier de 200 mm environ;
et optionnellement une couverture protectrice détachable pour les surfaces adhésives du support (1) et des bandes de contact (2a, 2b) etant en contact avec le corps du patient pendant usage.

2. Electrode selon la revendication 1, **caractérisée en ce que** les bandes de contact s'étendent, à l'exception de la region de passage aux pattes de connexion (1', 2a', 2b'), en substance parallèlement, écartées l'une de l'autre de 15 à 50 mm, de préférence de 20 à 40 mm, en particulier de 28 à 30 mm, ces bandes ayant une largeur de 3 à 10 mm, de préférence de 4 à 7 mm, en particulier de 5 à 6 mm.

3. Electrode selon la revendication 1 ou 2, **caractérisée en ce que**, à l'exception de la region des pattes de connexion (1', 2a', 2b'), la distance entre les bords extérieurs des bandes de contact (2a, 2b) et le bord extérieur du support (1) est de préférence de 3 et 15 mm, plus préférentiellement de 4 et 12 mm, la distance aux bords longitudinaux de l'électrode étant en particulier de 5 mm et celle au bout de l'électrode opposé aux pattes de connexion (1', 2a', 2b') étant en particulier de 10 mm.

4. Electrode selon l'une quelconque des revendications 1 à 3, **caractérisée en ce que** le support (1) est composé de mousse plastique non conductrice et adhésive sur une face.

5. Electrode selon la revendication 4, **caractérisée en ce que** l'adhésif sur l'une face du support (1) est non irritant également.

6. Electrode selon l'une quelconque des revendications précédentes, **caractérisée en ce que** la géométrie des pattes de connexion (1', 2a', 2b') correspond à celle des connexions éléctriques standardisées et commerciales, par exemple pour un borne de connexion des électrodes neutres utilisées dans la chirurgie haute fréquence.
